(19) Europäisches Patentamt
European Patent Office
Office européen des brevets

(11) Publication number: 0 187 130 A2

(12) EUROPEAN PATENT APPLICATION

(21) Application number: 85870194.9

(22) Date of filing: 23.12.85

(51) Int. Cl.⁴: C 07 C 99/00, C 07 C 101/12, C 07 C 101/20, C 07 C 101/30

(30) Priority: 28.12.84 US 687323

(43) Date of publication of application: 09.07.86 Bulletin 86/28

(84) Designated Contracting States: AT BE CH DE FR GB IT LI LU NL SE

(71) Applicant: Monsanto Company, Patent Department 800 North Lindbergh Boulevard, St. Louis Missouri 63167 (US)

(72) Inventor: Pulwer, Mitchell Joel, 13115 Old Farm drive, St. Louis Missouri 63146 (US)
Inventor: Balthazor, Terry Mack, 7208 Chamberlain Avenue, University City Missouri 63130 (US)
Inventor: Rogers, Thomas Edward, 409 Woodland Hill Court, Manchester Missouri 63011 (US)

(74) Representative: Lunt, John Cooper et al, Monsanto Europe S.A. Patent Department Avenue de Tervuren 270-272 Letter Box No 1, B-1150 Brussels (BE)

(54) **Process for producing N-substituted alpha-amino acids.**

(57) Secondary or tertiary alpha-amino acids are provided in high yield in one step by the reductive amination of glyoxylic acid in a reaction medium comprising glyoxylic acid, a primary or secondary amine, and water in an atmosphere of hydrogen using a palladium hydrogenation catalyst.

EP 0 187 130 A2

# PROCESS FOR PRODUCING N-SUBSTITUTED ALPHA-AMINO ACIDS

This invention relates to the production of N-substituted alpha-amino acids by the reductive amination of glyoxylic acid. More particularly, this invention relates to the reductive amination of glyoxylic acid with primary and secondary amines in the presence of a hydrogenation catalyst.

## Background of the Invention

The N-substituted alpha-amino acids provided by the process of this invention have a number of important and varied uses. Some of these compounds can be employed as buffering agents in pharmaceutical and cosmetic preparations. Also, many can be employed as key intermediates in the manufacture of peptides, plastics, polymers, and herbicides. Exemplary of an intermediate employed in the production of a herbicide is the preparation of N-phosphonomethylglycine through N-benzylglycine as described in U.S. Patent 3,835,000. Presently, the methods for preparing N-substituted alpha-amino acids are tedious and circuitous. The following are several representative examples whereby amino acids are provided by reductive amination. In U.S. 4,073,804 to Hearon, glyoxylic acid is reacted with ammonia to produce glycine utilizing a noble metal catalyst, in particular rhodium. Glyoxylic acid and ammonia are first reacted, and then the reaction product is transferred to a pressure vessel wherein hydrogenation of the reaction product takes place to provide glycine. The patent refers to an earlier process by Desnuelle et al, Bull. Soc. Chim., 5 1,700-2 (1934), Chem. Abstr., 28, col. 6,700 (1934). In the earlier procedure palladium is employed as a catalyst. As is pointed out by Hearon, the earlier process could not provide a reductive amination process with any hope for successful commercial application because the glycine product is obtained in

a yield of only 8% of the theoretical and can be isolated from the complex reaction mixture only in the form of a derivative.

A procedure for producing a glycine derivative by reductive amination is also disclosed by Skita and Wulff, Chem. Abstr., 21, col. 2876 (1927), wherein it is disclosed that the glycine derivative is provided in a two-step process. An amine is reacted with glyoxylic acid to provide an intermediate which is then hydrogenated under reductive amination conditions. Glycine is recovered in a multi-step isolation process by treating the glycine derivative produced by the two-step process. Recovery of the product from the crude reaction mixture is difficult.

There has been disclosed in the prior art processes for the production of N-substituted amino acids and in particular N-substituted glycines by the reaction of glycine with a hindered ketone in water in the presence of a noble metal catalyst. Such a process is disclosed in EPO Application 0 079 767 assigned to Exxon Research and Engineering Company. Reductive amination is carried out in the presence of an organic solvent to produce the N-substituted amino acid. The application also discloses carrying out the same reaction with unhindered aldehydes to produce a mixture of secondary and tertiary amino acids. Such a mixture is disclosed as being useful as a promoter in the "hot pot" acid gas scrubbing process. The reaction between the aldehyde and the amino acid results in a loss of water during the formation of the N-substituted amino acid.

Another method for providing N-substituted amino acids and in particular N-substituted glycine derivatives has been reported by Tien and Hunsberger in a series of publications during 1955. In the first publication entitled "A Phototropic Heterocyclic

Derivative of a Sydnone; A New Synthesis of an N-substituted Glycine; and a General Synthesis of Monosubstituted Hydrazines" appearing in Chemistry and Industry, January 29, 1955, they reported the production of the hydrochloride of N-(3-pyridyl)-glycine by mixing equimolar amounts of 3-aminopyridine and ethyl glyoxylate in water and then hydrogenating this solution in 6N hydrochloric acid in the presence of a palladium catalyst. In the J. Am. Chem. Soc., 77, 6604, (1955) further particular conditions for the above-described reaction providing N-(3-pyridyl)-glycine hydrochloride were provided. Finally Tien and Hunsberger reported in the J. Am. Chem. Soc., 77, 6696, (1955) the extension of the above-described process to provide N-(n-hexyl)-glycine and N-phenylglycine to demonstrate that their process for preparing N-substituted glycine from amines had very general application. However, in each instance glycine was only produced after treatment of an intermediate reaction product because the reaction only produced a glycine precursor.

N-substituted amino acids are produced according to a reaction disclosed in Japanese patent publication 57-156449 wherein an aldehyde acid or an alpha-keto acid is reacted with either ammonia, ammonium salts, or primary amines or primary amine salts to provide an N-acyl substituted amino acid in an aqueous solution. The reaction requires two moles of acid or ketone per mole of ammonium or amine and provides low yields.

According to U.K. Patent 1,514,402 saturated omega-amino acids are prepared by reductive amination of unsaturated aldehydoacids containing from 1 to 3 olefinic double bonds. The amino acid is obtained as an alkali metal salt by first treatment of an unsaturated aldehydoacid with an ammonium or an alkali

metal hydroxide in aqueous solution. This product is hydrogenated without isolation in a single stage in the presence of a catalyst to obtain the alkali metal salt of the desired saturated amino acid. Further treatment recovers the desired amino acid from the derivative produced by the above-described reaction.

## Summary of the Invention

There has now been discovered a novel process for the production of N-substituted alpha-amino acids by the catalytic hydrogenation of primary or secondary amines with glyoxylic acid to provide N-substituted alpha-amino acids directly in high yield and in one step. Surprisingly, dealkylation which has been the result of previous reductive amination processes employing primary amines does not occur, and the products obtained in accordance with the present invention are relatively pure and easily recovered.

The reaction is carried out in the presence of a proton accepting agent which can be merely a slight excess of the amine reactant. Also, the reaction provides a nearly quantitative yield of desired product in the presence of a hydrogenation catalyst selected preferably from palladium or iridium.

## Description of the Preferred Embodiments

In accordance with the present invention there has been discovered a novel process for the preparation of compounds represented by the formula

$$\begin{array}{ccccc} & R_1 & & O & \\ & | & & \| & \\ R- & N & -CH_2- & C & -OM \end{array}$$

wherein

R and $R_1$ are independently selected from the group consisting of hydrogen, alkyl, arylalkyl, $-CH_2-Y$ wherein Y is an electron withdrawing group,

$$\begin{array}{l} \quad\quad\;\; R_2 \\ \quad\quad\;\;\, | \\ -CH_2-CH-X \end{array}$$

wherein

X is a heteroatomic group or halogen, and

$R_2$ is selected from the group consisting of hydrogen, alkyl, and arylalkyl provided that only one of R and $R_1$ is hydrogen; and

M is selected from the group consisting of hydrogen and non-interfering salt-forming cations which comprises reacting an amine represented by the structure

$$\begin{array}{l} \quad\;\;\; R \\ \quad\;\;\;\, | \\ R_1-NH \end{array}$$

wherein R and $R_1$ are as defined above with compound represented by the structure

$$\begin{array}{l} \quad\; O\;\; O \\ \quad\; \|\;\;\; \| \\ H-C-C-OM \end{array}$$

wherein M is as defined above, in a reaction medium, in the presence of hydrogen, a catalytically effective amount of a hydrogenation catalyst and a proton acceptor.

The term "alkyl" as employed herein means straight, branched, and cycloalkyl groups having from 1 to 18 carbon atoms.

The term "arylalkyl" as employed herein means alkyl radicals substituted by at least one aryl group such as phenyl, naphthyl, and anthryl groups.

The term "heteroatomic group" means those functional groups which do not prevent the reductive amination reaction carried out in the process of this invention. That is, the group must not itself be capable of undergoing a hydrogenation reaction under the conditions that are required to provide the desired N-substituted alpha-amino acid. Such groups

include hydroxyl, alkoxy, thiol, thioalkyl, dialkylamino and the like.

The selection of the particular electron withdrawing group is well within the skill of the art and includes, but is not limited to, carboxyl, carboxymethyl, carboxyethyl, cyano, amido, thiocarboxy, sulfonyl, arylsulfonyl, sulfinyl and the like.

Non-interfering, salt-forming cations are, typically, cations of alkali metals, alkaline earth metals, ammonium, lower alkyl substituted phosphonium, and lower alkyl substituted sulfonium. Alkali metals include sodium, potassium and the like. Alkaline earth metals include magnesium and calcium and the like.

The reaction is preferably carried out in water as a solvent even in those instances wherein the amine reactant is only slightly soluble in water. Water is the preferred solvent because the end product is easily recovered and the troublesome disposal of organic solvents is avoided.

Surprisingly, the process of the present invention provides a much greater degree of control over the course of the reaction and thus over the product distribution than would be expected. Processes of the prior art employing primary amines provide mixtures including tertiary amines. See, for example, Rylander, "Catalytic Hydrogenation in Organic Synthesis", Academic Press, 1979. In prior art processes the rate of addition to the newly formed secondary amine was approximately the same as the rate of addition to the starting primary amine thereby providing, at best, a mixture of secondary and tertiary amines even in those processes wherein all the primary amine reacted. Presently, in accordance with this invention, the catalytic hydrogenation of a

1:1 and even a 1.25:1 mixture of primary amine to glyoxylic acid provides nearly quantitative conversion of the primary amine to N-substituted secondary alpha-amino acids. The surprising monoalkylation obtained in the present invention enables the production of highly pure secondary amino acids. However, if it is desired, a secondary amine can be employed as the starting material to provide a final product which is solely the tertiary N-substituted alpha-amino acid.

Because of the absence of tertiary amine in the reaction product resulting from the process of this invention, it is believed that the rate for producing monoalkylation is much faster than the reaction rate for providing the second addition-reduction reaction which provides the tertiary amine. However, the reaction rate is adequate for convenient production of the tertiary N-substituted alpha-amino acid from secondary amines if desired.

The process of this invention is carried out by placing the reactants in a pressure vessel equipped with a means for agitation. Typical means for agitation are, for example, a shaking apparatus or a stirrer or the like. Also, the vessel is conveniently equipped for the introduction of hydrogen gas and the regulation of pressure so that the desired pressure within the vessel is maintained. In most instances a pressure of from 100,000 to 500,000 $N/m^2$ is employed, and pressures to about 5,000,000 $N/m^2$ can be employed if so desired. The temperature employed in the process of this invention varies widely and is not critical. In most instances a convenient reaction temperature is in the range from about 15°C to about 85°C. Higher or lower temperatures can be employed but higher temperatures favor the formation of undesired by-products. As in most chemical processes

the reaction conditions, including temperature, must be adapted to suit the particular reactants employed and therefore the upper limit of reaction temperature is a function of the properties of the starting materials.

Because there is practically no production of side products in accordance with the present invention, the isolation of the desired product is a facile process. After completion of the reaction the catalyst is filtered from the mixture, and the solvent is removed providing the product in nearly quantitative yield. The proton acceptor can be removed by neutralization of the product mixture following removal of the catalyst.

Catalyst removal can be accomplished by a variety of means well known in the art. In addition to filtration, the product can be separated from the catalyst by recrystallization when a homogeneous catalyst is employed. In other cases it is possible to filter the catalyst and then remove the product from the reaction solvent by crystallization at low temperature. Yet in other instances the N-substituted alpha-amino acid is converted to a salt by means of a base, such as sodium hydroxide or potassium hydroxide or the like, and the product allowed to crystallize out of the reaction solvent. Also, the N-substituted alpha-amino acid may be obtained as the acid salt when an acid, such as hydrogen chloride or hydrogen bromide, is added to the reaction and the acid salt is crystallized.

Glyoxylic acid is known to exist as the hydrate of the aldehydic group under certain conditions. Also, glyoxylic acid may exist as a mixture of both the aldehyde and hydrate forms. When an alcoholic solvent is employed, the hemiacetal and acetal forms may exist. Any of the forms or the

mixture of the forms are suitable for use in the process of this invention. Thus, as employed herein the term "glyoxylic acid" means any of or a mixture of any of the forms described above.

The amines employed in the process of this invention may be selected from a wide variety of organic amines including primary or secondary alkylamines, alkylene amines and alkanol amines, such as methylamine, ethylamine, n-propylamine, isopropylamine, n-butylamine, isobutylamine, sec-butylamine, n-amylamine, isoamylamine, n-pentylamine, hexylamine, heptylamine, octylamine, nonylamine, decylamine, undecylamine, dodecylamine, tridecylamine, tetradecylamine, pentadecylamine, hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-n-propylamine, diisopropylamine, di-n-amylamine, diisoamylamine, dihexylamine, diheptylamine, dioctylamine, ethanolamine, n-propanolamine, isopropanolamine, diethanolamine, N-ethylpropanolamine, N-butylethanolamine, allylamine, n-butenyl-2-amine, n-pentenyl-2-amine, 2,3-dimethybutenyl-2-amine, di-buteny-2-amine, n-hexenyl-2-amine and propylenediamine; primary aryl amines, such as aniline, methoxyaniline, ethoxyaniline, o-, m-, or p-toluidine, phenylenediamine, benzidine, naphthylamine, o,m,p-chloroaniline and the like; heterocyclic amines, such as morpholine, piperidine, pyrrolidine and the like.

The above amines are exemplary only and are not intended to limit the invention in any way. Other organic nitrogen compounds which can be employed in the process of this invention include monoalkyl-

ammonium, dialkylammonium, monoalkenylammonium, dialkenylammonium, monoalkynylammonium, dialkynyl-ammonium, monoalkanolammonium, dialkanolammonium, heterocyclic ammonium or an aryl ammonium, such organic ammonium group containing from 1 to 18 carbon atoms.

In some instances the amine will form a salt of glyoxylic acid, and this can be used directly in the process of this invention even though such salt may, in certain instances, possess only slight solubility in water. Operable organic amines contain R and $R_1$ groups as defined above, bonded to the nitrogen atom which are not capable of undergoing a hydrogenation reaction under the conditions required in the process of this invention. However, the conditions employed in the process of this invention are relatively mild with respect to hydrogenation of R and $R_1$ groups. For example, the benzyl group is well known to be removed from amines by hydrogenation, but such amines can be employed in the process of the present invention to form N-benzylglycine.

Other proton acceptors which can be employed in the process of this invention, if it is not desirable to use a slight excess of the amine, include potassium hydroxide, sodium bicarbonate, potassium carbonate and the like. As mentioned above such bases can be removed at the conclusion of the hydrogenation reaction by neutralization with an acid, such as hydrochloric acid or sulfuric acid, and in some instances the corresponding salts may be employed in future reactions.

In addition to water as a reaction medium a variety of water-soluble organic solvents can be employed in conjunction with water in the process of this invention. Such mixtures are employed to maintain a solution of the reactants and products during the

course of the reaction. The use of water-soluble organic solvents is entirely optional and is not critical to the process of this invention since the amines employed usually have some solubility in water, even though slight, and will produce the desired product. For the purpose of increasing the rate of reaction it may be desirable to add a water-soluble organic solvent to the reaction mixture. These solvents are recovered from the reaction mixture and recycled. The solvents include, typically, lower aliphatic alcohols, such as methanol, ethanol, and isopropanol. In addition, other solvents include ethers, such as 1,4-dioxane and tetrahydrofuran, or the like. The amount of water-soluble organic solvent employed is not critical and may be adjusted to suit the particular conditions created by the selection of starting material.

The catalyst employed in the process of this invention is selected from the usual known hydrogenation catalysts which include those based on transition metals, such as nickel, osmium, palladium, platinum, rhodium, and ruthenium among others. These catalysts are preferably bound to a support, such as, for example, carbon and more particularly charcoal. Also, hydrogenation catalysts include the homogeneous type, such as hexarhodium hexadecacarbonyl, although such catalysts require further separation steps to remove the final product. Palladium is preferred because its use provides high yields.

The completion of the reaction is indicated by the amount of uptake of hydrogen gas or by NMR spectroscopy and other methods of analysis of the reaction mixture. The amount of hydrogen gas employed in the process of this invention depends upon the amount of reactants originally used. The amount of hydrogen uptake is usually very close to the

theoretical amount of hydrogen to satisfy the stoichiometry of the reaction. The time for completion of the reaction is dependent upon reaction conditions as, for example, concentration, solvent, temperature, amount and type of catalyst employed, and hydrogen pressure in the vessel. Normally the process of this invention is completed in a period of from 1 to 24 hours. Longer periods of time may be employed if required in any particular combination of reactants.

The following examples serve to further illustrate this invention and are not intended to limit the scope of the invention.

Example 1

To a flask equipped with a magnetic stirring bar there was added 14.8 g of a 50% aqueous solution of glyoxylic acid. 50 ml of water was added, and the flask was placed in an ice bath with stirring. Then 7.4 g of isopropylamine was added dropwise over 15 minutes. The solution was transferred to a Parr hydrogenator bottle which contained 2.0 g of 5% palladium on carbon. Hydrogen gas was introduced as the vessel was vented. This addition-venting procedure was carried out 3 times after which the vessel was closed and hydrogen gas introduced to give a pressure of about 300,000 $N/m^2$. The reaction proceeded with agitation provided by a shaker apparatus and was monitored by hydrogen uptake for 3 hours. The vessel was depressurized and the catalyst was removed by filtration. The catalyst was washed several times with water and the filtrates were combined. The solvent was removed under reduced pressure giving a solid which was analyzed by High Performance Liquid Chromatography and found to be pure N-isopropylglycine. To obtain the pure material as the hydrochloride salt, the solid was dissolved in water,

and then 10% hydrochloric acid was added until the pH of the solution was about 1. Then the solvent was removed under reduced pressure, and the solid which remained was recrystallized from acetic acid giving 14.7 g (96% yield) of a solid which was substantially pure N-isopropylglycine hydrochloride salt.

Example 2

The procedure of Example 1 was repeated with the exception that the product was not converted to the acid salt. The catalyst was removed and washed with water. The filtrates were combined and the solvent removed under reduced pressure until about 30 ml of solution remained. The solution was then purified by ion exchange chromatography giving 11.1 g (95% yield) of substantially pure N-isopropylglycine.

Example 3

The procedure in Example 1 was repeated with the exception that 2.0 g of 5% platinum on carbon was substituted for palladium on carbon. After 72 hours the yield of N-isopropylglycine was 56%.

Example 4

The procedure of Example 1 was repeated with the exception that 1.0 g of 10% rhodium on carbon was substituted for palladium on carbon. After 22 hours the yield of N-isopropylglycine was 49%.

Example 5

To a 15 L hydrogenator reactor was added 1777 g of a 50% aqueous solution of glyoxylic acid and then 3 L of water. To this was added a solution of 887 g of isopropylamine in 1 L of water over 30 minutes. Then 240 g of 5% palladium on carbon was added as a slurry in 1 L of water and the reactor was sealed and purged with helium gas. The reactor was then pressurized to about 1,800,000 $N/m^2$ of hydrogen and shaken at ambient temperature for 19 hours. The catalyst was filtered and washed with water, and the

filtrates were combined. The solvent was then removed under reduced pressure until only a small amount of the solvent remained. The solid was crystallized by treatment with a solution of acetone and isopropanol to give 1090 g (78%) of 97% pure N-isopropylglycine.

Example 6

In a flask was placed 14.8 g of a 50% aqueous solution of glyoxylic acid. To this, with stirring, was added 100 ml of water, and the flask was cooled to 5°C. Then 5.9 g of isopropylamine was added slowly after which the solution was transferred to a hydrogenator bottle. Then 2.0 g of 5% palladium on carbon was added along with 0.5 g of sodium hydroxide. The mixture was purged and then charged with 300,000 $N/m^2$ of hydrogen and shaken for 2 hours. The yield of N-isopropylglycine was 88%.

Example 7

Into a flask was placed 14.8 g of a 50% aqueous solution of glyoxylic acid. To this was added, with stirring, 100 ml of water, and the flask was cooled to 5°C. 5.9 g of isopropylamine was added slowly after which the solution was transferred to a hydrogenator bottle. Then 2.0 g of 5% palladium on carbon was added along with 1.0 g of sodium hydroxide. The mixture was purged and then charged with about 300,000 $N/m^2$ of hydrogen and shaken for 2 hours. The catalyst was filtered and washed with water. The yield of N-isopropylglycine was 92%. Another reaction mixture of the same quantities was prepared with the exception that previously used catalyst was employed instead of fresh catalyst. The catalyst was filtered and the yield of N-isopropylglycine was found to be 96%. The recovered catalyst was reused as in the above procedure again providing a yield of 89% of desired product in the second procedure.

Example 8

The procedure of Example 2 was repeated with the exception that 3.9 g of methylamine was substituted for isopropylamine. The yield of N-methylglycine was 74%.

Example 9

The procedure of Example 2 was repeated with the exception that 9.4 g of glycine was substituted for isopropylamine. The yield of iminodiacetic acid was 73%.

Example 10

The procedure of Example 2 was repeated with the exception that 12.4 g of cyclohexylamine was substituted for isopropylamine. The yield of N-cyclohexylglycine was 85%.

Example 11

The procedure of Example 2 was repeated with the exception that 5.6 g of ethylamine was substituted for isopropylamine. The yield of N-ethylglycine was 93%.

Example 12

The procedure of Example 2 was repeated with the exception that 9.1 g of diethylamine was substituted for isopropylamine. The reaction time was extended to 24 hours at which time there was found to be a 60% yield of N,N-diethylglycine.

Example 13

The procedure of Example 2 was repeated with the exception that 13.4 g of benzylamine was substituted for isopropylamine. The yield of N-benzylglycine was 89%.

Example 14

To a flask was added 72.3 g of a 50% aqueous solution of glyoxylic acid and 100 ml of water. The solution was stirred and cooled in an ice bath and then treated with a solution of 37.3 g of ethanolamine

in 100 ml of water. The solution was then transferred to a hydrogenator bottle, and 8.1 g of 5% palladium on carbon was added. The vessel was purged and then pressurized to about 400,000 $N/m^2$ of hydrogen. The reactor was shaken for 4 hours. The catalyst was then filtered from the reaction product and washed with water. The product was crystallized from the solution to give 52.4 g (90% yield) of N-(2-hydroxyethyl)-glycine.

Example 15

To a flask was added 44.5 g of beta-alanine, 74.0 g of a 50% aqueous solution of glyoxylic acid, and 150 ml of water. The mixture was transferred to an hydrogenator bottle, and then 10.0 g of 5% palladium on carbon was added. The vessel was purged several times using hydrogen and then pressurized to about 300,000 $N/m^2$ with hydrogen gas. The reaction was carried out for 22 hours, and then the catalyst was filtered and washed with hot water. The filtrates were combined and the solvent was removed giving a residue which was recrystallized from water/ethanol to give 35.8 g (78% yield) of N-carboxymethyl-beta-alanine.

Example 16

The procedure of Example 2 was repeated with the exception that the solvent used was 50 ml of a 1:1 mixture of methanol-water instead of water. The yield of N-isopropylglycine was 93%.

Example 17

The procedure of Example 6 was repeated with the exceptions that 1 g of 5% palladium on carbon was employed, and the temperature of the reaction was 50°C. The yield of N-isopropylglycine was 91%.

Example 18

The procedure of Example 17 was repeated with the exception that the temperature of the

reaction was 80°C. The yield of N-isopropylglycine was 94%.

Example 19

The procedure of Example 18 was repeated with the exception that 0.5 g of 5% palladium on carbon was used. The yield of N-isopropylglycine was 91%.

Example 20

In a 500 ml hydrogenation bottle was placed 14.8 g of a 50% aqueous solution of glyoxylic acid. To this, with stirring, was added 50 ml of water and 2.0 g of 50% NaOH. The flask was cooled to 5°C. Then 8.7 g of n-pentylamine was added slowly with agitation. Then 2.0 g of 5% palladium on carbon was added. The mixture was purged and then pressurized to 300,000 $N/m^2$ of hydrogen and shaken at 40°C for 3 hours. The yield of n-pentylglycine was 10.5 g (72.4%).

Although this invention has been described with respect to specific modifications, the details thereof are not to be construed as limitations for it will be apparent that various equivalents, changes, and modifications may be resorted to without departing from the spirit and scope thereof, and it is understood that such equivalent embodiments are intended to be included herein.

WHAT IS CLAIMED IS:

1. A process for preparing N-substituted alpha-amino acids and derivatives thereof represented by the structure

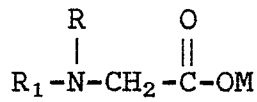

I

wherein

R and $R_1$ are selected from the group consisting of hydrogen, alkyl, arylalkyl, $-CH_2-Y$ wherein Y is an electron withdrawing group,

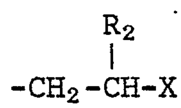

II

wherein

X is a heteroatomic group or halogen, and

$R_2$ is selected from the group consisting of hydrogen, alkyl, and arylalkyl provided that only one of R and $R_1$ is hydrogen; and

M is selected from the group consisting of hydrogen and non-interfering salt-forming cations which comprises reacting an amine represented by the structure

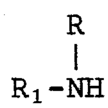

III

wherein R and $R_1$ are as defined above with a glyoxylate represented by the structure

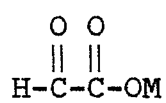

IV

wherein M is as defined above, in a reaction medium, in the presence of hydrogen, a catalytically effective amount of a hydrogenation catalyst and a proton acceptor.

2. A process of Claim 1 wherein $R_1$ is hydrogen and R is alkyl.

3. A process of Claim 2 wherein $R_1$ is isopropyl.

4. A process of Claim 2 wherein $R_1$ is n-pentyl.

5. A process of Claim 2 wherein M is hydrogen.

6. A process of Claim 5 wherein the hydrogen catalyst is palladium bound to a support.

7. A process of Claim 6 wherein the reaction medium is water.

8. A process of Claim 1 wherein R is arylalkyl and $R_1$ is hydrogen.

9. A process of Claim 7 wherein R is benzyl.

10. A process of Claim 9 wherein M is hydrogen.

11. A process of Claim 1 wherein R is

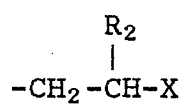

$R_1$ is hydrogen and M is hydrogen.

12. A process of Claim 11 wherein X is a hydroxyl group and $R_2$ is hydrogen.

13. A process of Claim 1 wherein R is $-CH_2-Y$, $R_1$ is hydrogen, and M is hydrogen.

14. A process of Claim 13 wherein Y is carboxyl.

15. A process of Claim 1 wherein M is a salt-forming cation.

16. A process of Claim 1 wherein the proton acceptor is the amine reactant.

17. A process of Claim 1 wherein the proton acceptor is an alkali metal hydroxide.

18. A process of Claim 17 wherein the proton acceptor is sodium hydroxide.

19. A process of Claim 17 wherein the mole ratio of III to IV is about 1:1.

20. A process of Claim 1 wherein the compound of Formula III is in aqueous solution.

21. A process of Claim 1 wherein the temperature is maintained within the range of from about 15°C to about 85°C.

22. A process of Claim 1 wherein the hydrogenation catalyst is selected from the group consisting of nickel, osmium, palladium, platinum, iridium, rhodium, and ruthenium.

23. A process of Claim 7 wherein the hydrogenation catalyst is on a carbon support.

24. The process of Claim 1 wherein R and $R_1$ are alkyl and M is hydrogen.

25. The process of Claim 1 wherein M is hydrogen.

26. The process of Claim 25 wherein the compound of Formula IV is in the form of its hydrate.

27. The process of Claim 1 wherein the reaction medium is selected from the group consisting of water or a mixture of water and a water soluble organic solvent.

28. The process of Claim 27 wherein the organic solvent is selected from the group consisting of methanol, ethanol, and tetrahydrofuran.

29. A process which comprises reacting an aqueous solution of glyoxylic acid with isopropylamine and hydrogenating the reaction mixture in the presence of palladium on a support of carbon.

30. A process of Claim 1 wherein the proton acceptor is an alkali metal carbonate.

31. A process of Claim 1 wherein R is ethyl.